# EUROPEAN PATENT SPECIFICATION

(11) **EP 1 267 886 B1**
(45) Date of publication and mention of the grant of the patent: **08.03.2006**
(21) Application number: 01920583.0
(22) Date of filing: 20.03.2001
(51) Int. Cl.: A61K 31/59, A61P 37/06, A61P 13/12

(54) **VITAMIN D COMPOUNDS FOR THE TREATMENT OF CHRONIC ALLOGRAFT NEPHROPATHY IN KIDNEY TRANSPLANT PATIENTS**
VITAMIN D-VERBINDUNGEN ZUR BEHANDLUNG VON CHRONISCHER TRANPLANTATNEPHROPATHIE BEI NIEREN TRANSPLANTIEREN PATIENTEN
COMPOSES DE VITAMINE D UTILISES POUR LE TRAITEMENT DE LA NEPHROPATHIE CHRONIQUE DE L'ALLOGREFFE CHEZ DES PATIENTS AYANT SUBI UNE TRANSPLANTATION RENALE

(30) Priority: 27.03.2000 US 192649 P
(43) Date of publication of application: 02.01.2003
(73) Proprietor: WISCONSIN ALUMNI RESEARCH FOUNDATION, Madison, WI 53705 (US)
(72) Inventor: DELUCA, Hector, F., Deerfield, WI 53531 (US); BECKER, Bryan, N., Madison, WI 53717 (US); SOLLINGER, Hans, W., Madison, WI 53703 (US); HULLETT, Debra, A., Madison, WI 53711 (US)
(74) Representative: Ellis-Jones, Patrick George Armine
(86) International application number: PCT/US2001/008939
(87) International publication number: WO 2001/072292

(56) References cited:
- WO-A-98/18468
- WO-A-98/55127
- WO-A-99/12894
- WO-A-99/18070
- E.KALLIO E.A.: "MC1288, a vitamin D analogue, reduces short- and long-term renal allograft rejection in the rat" TRANSPLANTATION PROCEEDINGS, vol. 28, no. 6, 1996, page 3113 XP001052993
- R.PEREZ CALDERON: "Calcitriol induces apoptosis of incubated lymphocyte T cells from patients with acute renal graft rejection " TRANSPLANTATION PROCEEDINGS, vol. 31, no. 6, 1999, pages 2311-2313, XP001052974

## Description

Chronic rejection is the major cause of failure of kidney transplants, other than patient death. Chronic allograft nephropathy (CAN) is characterized by functional impairment of the kidney and has a pathology including tubular atrophy, interstitial fibrosis, and fibrous intimal thickening. Factors involved may include pre-existing chronic conditions in the donor, acute injury related to the transplant process, and immune stress. One indication of CAN is a changing serum creatinine level. Up to 40% percent of kidney grafts develop progressive dysfunction, despite the use of immunosuppressive drugs (L.C. Paul, Kidney International 56:783-793, 1999).

Standard immunosuppressive drug therapy includes cyclosporine A, tacrolimus and corticosteroids. Additional immunosuppressive therapies include azathioprine, mycophenolate mofetil, sirolimus, rapamycin, rapamycin analogs and prednisone.

One focus of transplant research today is to reduce the amount of immunosuppresive drug usage after kidney transplantation. Cyclosporine-treated patients are known to develop nephrotoxicity and hypertension. Diabetes mellitus occurs in approximately 15% of renal transplant patients. Additionally, immunosuppressive drug have negative cosmetic side effects.

Needed in the art of renal transplantation is an improved therapy for stabilizing kidney function after transplantation and lowering the amount of immunosuppresive therapy needed for a stabilized kidney transplant.

The invention provides use of a vitamin D compound in the preparation of a medicament for the treatment of chronic allograft nephropathy in a kidney transplant patient, wherein the transplant patient is undergoing immunosuppressive therapy. Kidney transplant patients who are receiving immunosuppressive therapy are provided with a sufficient amount of a vitamin D compound wherein kidney function stabilizes or rate of loss of kidney function decelerates. Kidney function is preferably measured by serum creatinine levels.

In a preferred method of the present invention, the vitamin D compound is 1,25-dihydroxy vitamin D₃ and the treatment method is oral delivery.

It is an object of the present invention to stabilize kidney function after kidney transplant.

It is another object of the present invention to decelerate loss of kidney function after a kidney transplant.

It is an advantage of the present invention that this stabilization or deceleration of loss of function takes place in the presence of standard immunosuppressive therapy.

Other objects, features and advantages of the present invention will become apparent to one of skill in the art after review of the specification in claims.

### A. In General

Renal transplantation is the most common form of solid organ transplantation in the United States. Interestingly, patients entering into renal transplantation frequently have aberrant regulation of their vitamin D hormonal axis as a consequence of renal failure. Vitamin D production declines early in the setting of renal insufficiency and vitamin D supplementation is required in many end-stage renal disease (ESRD) patients to stabilize parathyroid gland function and calcium status.

Small clinical studies suggest that early after renal transplantation, mild-to-moderate vitamin D deficiency may still exist in up to 40% of patients (P.I. Lobo, et al., Clin. Transplant 9[4]:277-281, 1995; R. Carter, et al., Transplantation 67:S168, 1999). This may be due to abnormalities in vitamin D metabolism inherent in the new allograft, e.g. depressed renal transplant function and unrecognized renal epithelial cell damage. These are manifested by elevated serum creatinine values. (P.I. Lobo, et al., supra, 1995; R. Carter, et al., supra, 1999) The consequences of even relative vitamin D deficiency in this setting have not been examined extensively in the transplant population.

Another aspect of vitamin D activity that may be significantly affected by aberrant vitamin D production and metabolism is the potential immunosuppressive effects associated with vitamin D. Skin (P. Veyron, et al., Transplant Immunol. 1:72-76, 1993), heart (J.M. Lemire, et al., Transplantation 54:762-763, 1992), and kidney transplant (E. Lewin and K. Olgaard, Calcif. Tissue Int. 54:150-154, 1994; M.T. Cantorna, Transplantation 66(7):828-831, 1998; D.A. Hullett, et al., Transplantation 66(7):824-828, 1998) survival have all been prolonged by the administration of various vitamin D compounds.

### B. Investigation of Calcitriol as a Transplant Therapy

We were interested in determining whether vitamin D compounds, preferably the most common vitamin D supplement 1,25-dihydroxyvitamin D₃ (calcitriol), exerted beneficial effect on renal transplant function. To this end we examined all patients who received calcitriol following kidney or kidney-pancreas transplantation at the University of Wisconsin to determine whether the administration of calcitriol was associated with a change in transplant function. The examples below demonstrate that we found that there were no adverse events identified in association with vitamin D compound therapy and that vitamin D therapy appears to be beneficial in preserving renal graft function in the setting of kidney or kidney-pancreas transplantation. The introduction of the vitamin D compound into a transplant course with declining renal function was associated with stabilization of and preservation of renal function along with a significant deceleration in the rate of loss of function. Calcitriol used early in the post transplant setting, during the treatment period with the largest dosages of calcineurin inhibitors, was also associated with stable renal graft function without any noted loss of function.

In one embodiment, the present invention is treatment of a kidney transplant with an effective amount of a vitamin D compound, wherein the patient is also treated with immunosuppressant. By "kidney transplant" patient, we mean to include all patients who have had kidney transplants or kidney pancreas transplants.

In a particularly advantageous form of the reaction, the administered compound is either 1α,25-dihydroxyvitamin D₃ (1,25-(OH)₂D₃), 19-nor 1,25-dihydroxyvitamin D₂ (1 9-nor-1,25-(OH)₂D₃), 24-homo-22-dehydro-22E-1α,25-dihydroxyvitamin D₃ (24-homo-22-dehydro-22E-1,25-(OH)₂D₃), 1,25-dihydroxy-24(E)-dehydro-24-homo-vitamin D₃ (1,25-(OH)₂-24-homo D₃), or 19-nor-1,25-dihydroxy-21-epi-vitamin D₃ (19-nor-1,25-(OH)₂-21-epi-D₃).

In another form of the present invention, the vitamin D compound has the formula 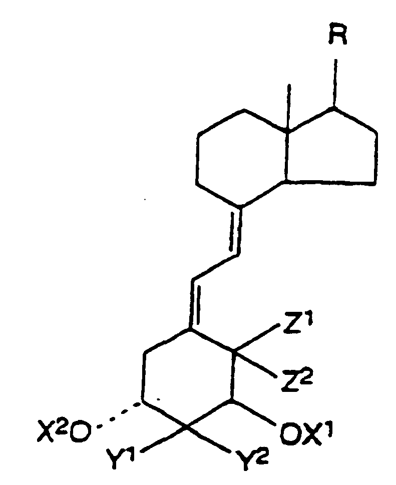
wherein X¹ and X² are each chosen from hydrogen and acyl; wherein Y¹ and Y² can be H, or one can be 0-aryl, 0-alkyl, aryl, C₁₋₄ alkyl, taken together to form an alkene having the structure of 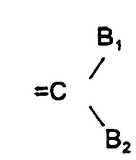
where B₁ and B₂ can be chosen from H, C₁₋₄ alkyl and aryl, and can have a β or α configuration; Z¹=Z²=H or Z¹ and Z² together are =CH₂; and wherein R is an alkyl, hydroxyalkyl or fluoroalkyl group, or R may represent the following side chain: 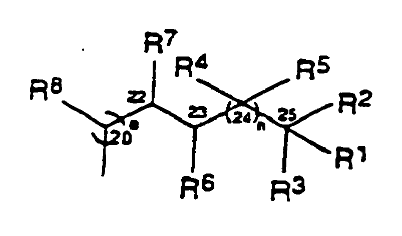
wherein (a) may have an S or R configuration, R¹ represents hydrogen, hydroxy or O-acyl, R² and R³ are each chosen from alkyl, hydroxyalkyl and fluoralkyl, or, when taken together represent the group-(CH₂)ₘ-wherein m is an integer of from 2 to 5, R⁴ is chosen from hydrogen, hydroxy, fluorine, O-acyl, alkyl, hydroxyalkyl and fluoralkyl, wherein if R⁵ is hydroxyl or fluoro, R⁴ must be hydrogen or alkyl, R⁵ is chosen from hydrogen, hydroxy, fluorine, alkyl, hydroxyalkyl and fluoroalkyl, or R⁴ and R⁵ taken together represent double-bonded oxygen. R⁶ and R⁷ taken together form a carbon-carbon double bond, R⁸ may be H or CH₃, and wherein n is an integer of from 1 to 5, and wherein the carbon at any one of positions 20, 22, or 23 in the side chain may be replaced by an O, S, or N atom.

One may evaluate a candidate vitamin D compound for its suitability for the present invention. The candidate compound may first be subjected to an initial mouse-model screening procedure. A successful compound will result in stabilized kidney function or a deceleration in kidney function lost, preferably to the extent shown in the Examples for 1,25-(OH)₂D₃. However, a successful compound is generally described as one that stabilizes a patient's serum creatinine levels. The patient should show a significant stabilization of serum creatinine, preferably wherein the level is <3.0 mg/dl, most preferably <2.6 mg/dl, for at least 500 days after transplant.

In another version of the present invention, the rise in serum creatinine levels (indicative of declining renal function) will be reduced compared to patients not treated with vitamin D compound. In one preferred embodiment, the patient's serum creatinine level will not rise more than 1.5 mg/dl compared to level prior to transplant for a period of 500 days after transplant. In another preferred embodiment, the level will not double within 500 days after transplant.

A preferred dose of vitamin D compound for the present invention is the maximum that a patient can tolerate and not develop hypercalcemia. If the vitamin D compound is calcitriol, a particularly advantageous daily dose of the compound is between 0.50 and 0.75 µg per day per 72.6 kg (160 pound) patient. In general, the preferred dose of vitamin D compound is between 0.1 and 50 µg per day per 72.6 kg (160 pound) patient.

1,25-dihydroxyvitamin D₃ (1,25-(OH)₂D₃) is currently administered at a level of 0.5 µg/day per 72.6 kg (160 pound) patient, usually in two quarter microgram capsules morning and night, for the treatment of osteoporosis or renal osteodystrophy.

Therefore, the preferred dose of 1,25-(OH)₂D₃ would appear to be from 0.5 to 0.75 µg/day. Other less active 1α-hydroxy vitamin D compounds can be given at higher doses safely. For example, in Japan the treatment of osteoporosis with 1,25-(OH)₂D₃ is from 0.5 to 1.0 µg/day. The same is true of other countries, such as Italy, where as much as 1 µg/day of 1,25-(OH)₂D₃ has been successfully used by Dr. Caniggia (A. Caniggia, et al., Metabolism 39:43-49, 1990).

A preferred mode of treatment is daily, oral administration, preferably with a slow release formulation or a slow release compound. The dose is preferably oral, but could be administered in other manners, such as by injection. Applicants specifically envision that a fairly continuous dosing of vitamin D compound is advantageous in reduction of SLE disease symptoms.

The Examples below describe preferable vitamin D compound dosages ranging between 0.5 µg weekly to 0.75 µg daily.

One would preferably evaluate renal function by assessing serum creatinine values, preferably as described below in the Examples. The Examples below disclose that the mean serum creatinine levels increase in patients with no vitamin D compound treatment, thus indicating declining renal function. The data indicate that once calcitriol therapy was initiated, the serum creatinine level stabilized. We define a "stabilized" level as a level <3.0 mg/dl for a period of 500 days after transplant. A "deceleration in kidney function loss" is defined as slowing the rate of loss of renal function as reflected by no change or minimal fluctuation in serum creatinine values or in other measures of renal function such as creatinine clearance or glomerular filtration rate. We expect a serum creatinine level change of less than 1.5 mg/dl/500 days after transplant.

Vitamin D therapy would preferably begin immediately after transplantation or at some point further along in the clinical course after transplantation.

A suitable patient has had a kidney or kidney-pancreas transplant and has received standard transplant rejection therapies, such as administration of cydosporine A. Typical immunosuppressive therapy would include: corticosteroids, cyclosporine A or tacrolimus, mycophenolate mofetil and/or use of rapamycin or rapamycin analogs and/or azathioprine.

### EXAMPLES

We examined all patients who received calcitriol following kidney or kidney-pancreas transplantation at the University of Wisconsin to determine whether the administration of calcitriol was associated with a change in transplant function.
Methodology-retrospective analysis: The University of Wisconsin transplant database was screened to identify any kidney and/or kidney-pancreas transplant recipient who received 1,25-dihydroxyvitamin D₃ (calcitriol) peri - post-transplant. Clinical and demographic variables were abstracted from the database. Those patients with adequate follow-up data were included in the analysis (≥ 1 year follow-up data following the initiation of calcitriol). Demographic variables included race, age, and any history of parathyroidectomy as these patients would likely be receiving calcitriol at time of transplantation. The effect of calcitriol treatment on renal function was analyzed using general linear mixed modeling of the change in slope of renal function prior to and following the start of calcitriol therapy. The effect of calcitriol on cyclosporine A (CsA) and tacrolimus (FK506) serum levels was analyzed by standardizing the milligram dosages of these agents to a mean of 0 with a standard deviation of 1. Adverse events and hypercalcemia were noted by identifiers in the database and by serum calcium levels as recorded in the database. Hypercalcemia was defined as a serum calcium > 10.5 mg/dl.
Data assessment-Demographics: Calcitriol-treated patients were divided into:
Group 1: patients who initiated calcitriol therapy > one year following transplantation.
Group 2: patients who remained on or initiated calcitriol therapy within two weeks of transplantation.
(A third group of patients were also identified. These patients were started on calcitriol therapy between 15 days and 1 year following transplantation. These patients have not been completely evaluated at this point in time.)

The demographic characteristics for Group 1 and Group 2 patients are shown in Table 1. When appropriate, these data are represented as mean ± standard deviation. The vast majority of patients in both groups were caucasian.

**Table 1. Demographics for calcitriol-treated patients**

| **Characteristic** | **Group 1** | **Group 2** |
|---|---|---|
| No. | 26 | 22 |
| Average age at Tx (yrs) | 41.3±11.8 | 46.5±14.5 |
| M/F | 16/10 | 13/9 |
| Caucasian/African-American/Other | 24/1/1 | 18/3/1 |
| Donor age* | 31±16.7 | 28.6±14 |
| Cadaver/live donor Tx** | 15/9 | -- |
| Pre-Tx parathyroidectomy | 5 | 3 |
| *donor age available for n=15 in Group 1; **data available for n=24 in Group 1, not available for Group 2 patients | | |

Type 1 diabetes mellitus was the most common cause or etiology of end-stage renal disease (ESRD) in Group 1 patients (n=9) (Table 2). A variety of other etiologies accounted for ESRD in the remaining patients. All of these entities also occurred in the Group 2 patients though with a different prevalence (Table 2).

**Table 2. Etiologies for ESRD in Group 1 and Group 2 calcitriol-treated patients**

| **Cause of ESRD** | **Group 1** | **Group 2** |
|---|---|---|
| Type 1 diabetes mellitus | 9 | 2 |
| Chronic glomerulonephritus | 1 | 2 |
| Hypertension | 3 | 2 |
| Focal segmental glomerulosclerosis | 3 | 1 |
| IgA nephropathy | 1 | 2 |
| Membranous glomerulonephritis | 3 | 1 |
| Other | 6 | 12 |

Calcitriol dosages ranged between 0.5 µg weekly to 0.75 µg daily, with no significant difference between dosage ranges between Group 1 and Group 2.
Data assessment-outcomes for Group 1 patients: Renal function was determined by assessing serial serum creatinine values. This serum measure is a standard and accepted measure of renal function. The start of calcitriol therapy was defined as day o. Time prior to initiation of calcitriol therapy was designated by a negative value. Mean serum creatinine levels appeared to increase in Group 1 patients (indicative of declining renal function) until the time of calcitriol therapy was initiated (Table 3).

**Table 3. Serum creatinine values in Group 1 patients prior to and following initiation of calcitriol therapy.**

| **Day** | **Mean serum creatinine (mg/dl)** | **Standard deviation** |
|---|---|---|
| -500 | 1.39 | 0.52 |
| -400 | 1.52 | 0.48 |
| -300 | 1.68 | 1.23 |
| -200 | 1.91 | 1.09 |
| -100 | 2.65 | 1.44 |

| **Therapy Administered** | | |
|---|---|---|
| 100 (calcitriol) | 2.54 | 2.26 |
| 200 (calcitriol) | 2.44 | 1.26 |
| 300 (calcitriol) | 2.50 | 1.21 |
| 400 (calcitriol) | 2.27 | 1.34 |
| 500 (calcitriol) | 2.37 | 1.47 |

Serum creatinine levels stabilized following initiation of calcitriol therapy. These results were substantiated by a repeated measures analysis of variance of the slopes of creatinine trends over time. This analysis indicated that the rate of increase in serum creatinine was greatest in the interval immediately pre-calcitriol therapy (0.007 mg/day) (p=0.009 vs. calcitriol therapy period). After 300 days of calcitriol therapy, creatinine was decreasing evidenced by a negative slope, suggesting a significant stabilization or deceleration of the rate of loss of renal graft function with therapy. This is demonstrated in Table 4 in which "difference" is defined as the change in slope of a patient's serum creatinine plotted over time. A negative value for this slope denoted a decreasing slope and improved renal function.

**Table 4. Change in slope of creatinine over time in Group 1 patients.**

| **Treatment day** | **difference** | **P value** |
|---|---|---|
| +100 | -0.0050 | 0.128 |
| +200 | -0.0046 | 0.187 |
| +300 | -0.0080 | 0.031 |
| +400 | -0.0085 | 0.031 |
| +500 | -0.0078 | 0.041 |

Ultimately, six patients in this group had graft failures (loss of the transplant).
Data assessment-outcomes for Group 2 patients: The start of calcitriol therapy was again defined as day 0. Mean serum creatinine levels remained stable in this patient cohort for the first 600 days following initiation of calcitriol (Table 5).

**Table 5. Serum creatinine values in Group 1 patients prior to and following initiation of calcitriol therapy**

| **Day** | **Mean serum creatinine (mg/dl)** | **Standard deviation** |
|---|---|---|
| 100 (calcitriol) | 1.61 | 0.65 |
| 200 (calcitriol) | 1.79 | 0.50 |
| 300 (calcitriol) | 1.70 | 0.37 |
| 400 (calcitriol) | 1.62 | 0.44 |
| 500 (calcitriol) | 1.82 | 0.49 |

The analysis of slopes found no interview slopes significantly different from 0 nor any interval slopes significantly different from one another. These data suggested that calcitriol therapy was associated with stabilization of early renal graft function in the setting of kidney and kidney-pancreas transplantation.

Ultimately, there were two graft failures in this group.
Data assessment-effect of calcitriol on immunosuppressive agents: The calcineurin inhibitors, cyclosporine A (CsA) and tacrolimus (FK506) are standard immunosuppressive agents for kidney and kidney-pancreas transplant recipients. Both of these agents have beneficial effects in prolonging allograft function by altering the ability for activated T cells to produce interleukin-2 (IL-2). However, both of these agents are also associated with drug-related nephrotoxicity that is manifested by characteristic histologic changes in the allograft, e.g. tubulointerstitial fibrosis, and loss of allograft function long-term. Thus, it was important to note in this retrospective analysis whether calcitriol altered CsA or FK506 serum levels, potentially altering their immunosuppressive effects on the immune system and their potential long-term nephrotoxic effects.

Seven of the 26 Group 1 patients had serial CsA levels following the initiation of calcitriol therapy. No trends in either mean CsA levels or variability in CsA levels were noted.

To make the calcineurin inhibitors relatively comparable for the purposes of statistical analyses, the milligram dosages for each were standardized to a mean of 0 and a standard deviation of 1. All of the Group 2 patients were treated either with CsA or FK506 therefore they were analyzed in combination with the Group 1 CsA-treated patients to determine the effect of calcitriol, if any, on drug dosing requirements. Calcitriol therapy had no significant effect on CsA or FK506 dosages or serum drug levels in a combined analysis of all Group 1 and Group 2 patients and there were no trends influenced by calcitriol in CsA or FK506 dosage requirements or serum drug levels.
Data assessment-calcitriol and adverse events: There were no adverse events identified in association with calcitriol therapy. The mean serum calcium in Group 1 and Group 2 patients was < 10 mg/dl (9.6±0.9 mg/dl). There were no episodes of sustained hypercalcemia (>2 serial hypercalcemic values noted) or hypercalcemic events requiring hospitalization. There were no episodes of sustained hematuria that could be directly attributable to hypercalcemia.
Summary-calcitriol therapy in kidney and kidney-pancreas transplantation: Calcitriol therapy appears to be beneficial in preserving renal graft function in the setting of kidney or kidney-pancreas transplantation as determined in this retrospective study. The introduction of calcitriol into a transplant course with declining renal function was associated with stabilization of and preservation of renal function along with a significant deceleration in the rate of loss of function. Calcitriol use early in the post-transplant setting, during the treatment period with the largest dosages of calcineurin inhibitors, also was associated with stable renal graft function without any noted loss of function.

## Claims

1. Use of a vitamin D compound in the preparation of a medicament for the treatment of chronic allograft nephropathy in a kidney transplant patient, wherein the transplant patient is undergoing immunosuppressive therapy.

2. The use according to claim 1, wherein the vitamin D compound is 1, 25-dihydroxyvitamin D₃.

3. The use according to claim 1, wherein the vitamin D compound is a 1α-hydroxy compound.

4. The use according to claim 1, wherein the amount of vitamin D compound administered is between 0.1 µg and 50 µg per day per 72.6 kg (160 pound) patient.

5. The use according to claim 4, wherein the amount of vitamin D compound administered is between 0.1 µg and 0.75 µg per day per 72.6 kg (160 pound) patient.

6. The use according to claim 1 or 2, wherein the vitamin D compound suitable for administration orally and daily.

7. The use according to claim 1, wherein the administration of the vitamin D compound begins within 24 hours of the kidney transplant procedure.

8. The use according to claim 1, wherein the administration of vitamin D compound begins before the kidney transplant procedure.

9. The use according to claim 1, wherein the vitamin D compound is of the following formula: 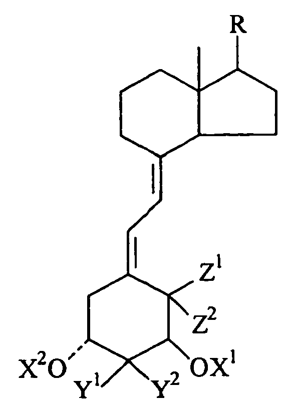
wherein X¹ and X² are each chosen from hydrogen and acyl:
wherein Y¹ and Y² can be H, or one can be O-aryl, O-alkyl, aryl, alkyl of 1-4 carbons, taken together to form an alkene having the structure of 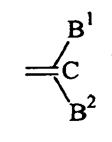
where B₁ and B₂ can be chosen from H, alkyl of
1-4 carbons and aryl or alkyl can have a β or α configuration;
Z¹=Z²=H or Z¹ and Z² together are =CH₂; and
wherein R is an alkyl, hydroxyalkyl or fluoroalkyl group, or R may represent the following side chain: 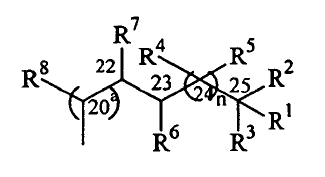
wherein (a) may have an S or R configuration, R¹ represents hydrogen, hydroxy or O-acyl, R² and R³ are each chosen from alkyl, hydroxyalkyl and fluoralkyl, or, when taken together represent the group-(CH₂)ₘ- wherein m is an integer having a value of from 2 to 5, R⁴ is chosen from hydrogen, hydroxy, fluorine, O-acyl, alkyl, hydroxyalkyl and fluoroalkyl, wherein if R⁵ is hydroxy or fluoro, R⁴ must be hydrogen or alkyl, R⁵ is chosen from hydrogen, hydroxy, fluorine, alkyl, hydroxyalkyl and fluoroalkyl, or R⁴ and R⁵ taken together represent double-bonded oxygen, R⁶ and R⁷ taken together form a carbon-carbon double bond, R⁸ may be H or CH₃, and wherein n is an integer having a value of from 1 to 5, and wherein the carbon at any one of positions 20, 22 or 23 in the side chain may be replaced by an O, S or N atom.

10. The use according to claim 9, wherein the compound is chosen from 1, 25-dihydroxyvitamin D₃, 19-nor-1,25-dihydroxyvitamin D₂, 19-nor-21-epi-1,25-dihydroxyvitamin D₃, 1,25-dihydroxy-24-homo-22-dehydro-22E vitamin D₃, and 19-nor-1,25-dihydroxy-24-homo-22-dehydro-22E-vitamin D₃.

## Revendications

1. Utilisation d'un composé du type vitamine D dans la préparation d'un médicament destiné au traitement de la néphropathie chronique due à une allogreffe chez un patient ayant subi une transplantation rénale, qui est soumis à une thérapie immunosuppressive.

2. Utilisation selon la revendication 1, dans laquelle le composé du type vitamine D est la 1,25-dihydroxyvitamine D₃.

3. Utilisation selon la revendication 1, dans laquelle le composé du type vitamine D est un composé 1α-hydroxylé.

4. Utilisation selon la revendication 1, dans laquelle la dose administrée de composé du type vitamine D est comprise entre 0,1 µg et 50 µg par jour, pour un patient de 72,6 kg (160 livres).

5. Utilisation selon la revendication 4, dans laquelle la dose administrée de composé du type vitamine D est comprise entre 0,1 µg et 0,75 µg par jour, pour un patient de 72,6 kg (160 livres).

6. Utilisation selon la revendication 1 ou 2, dans laquelle le composé du type vitamine D est un composé convenant à une administration journalière par voie orale.

7. Utilisation selon la revendication 1, dans laquelle l'administration du composé du type vitamine D commence dans les 24 heures après l'opération de transplantation rénale.

8. Utilisation selon la revendication 1, dans laquelle l'administration du composé du type vitamine D commence avant l'opération de transplantation rénale.

9. Utilisation selon la revendication 1, dans laquelle le composé du type vitamine D est un composé répondant à la formule suivante : 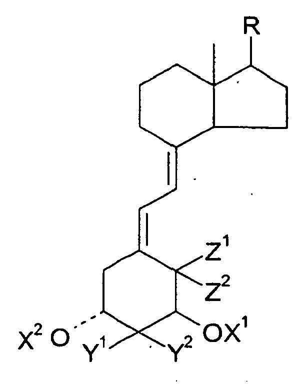
dans laquelle X¹ et X² représentent chacun un atome d'hydrogène ou un groupe acyle, Y¹ et Y² peuvent être des atomes d'hydrogène, ou bien l'un peut être un groupe O-aryle, O-alkyle, aryle ou alkyle ayant 1 à 4 atomes de carbone, ou bien ils peuvent former ensemble un groupe alkylidène de structure 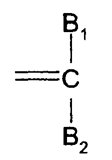
dans laquelle B₁ et B₂ peuvent être un atome d'hydrogène, un groupe alkyle ayant 1 à 4 atomes de carbone ou un groupe aryle, et peuvent avoir une configuration αou β, Z¹ et Z² représentent des atomes d'hydrogène ou bien Z¹ et Z² forment ensemble un groupe =CH₂, et R représente un groupe alkyle, un groupe hydroxyalkyle ou un groupe fluoroalkyle, ou la chaîne latérale suivante : 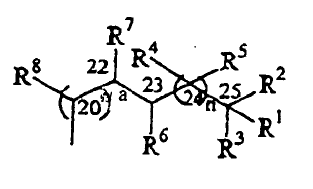
dans laquelle (a) peut avoir une configuration S ou R, R¹ représente un atome d'hydrogène ou un groupe hydroxyle ou O-acyle, R² et R³ représentent chacun un groupe alkyle, hydroxyalkyle ou fluoroalkyle, ou bien, pris ensemble, représentent le groupe -(CH₂)ₘ- où m est un nombre entier de 2 à 5, R⁴ représente un atome d'hydrogène ou de fluor, ou un groupe hydroxyle, O-acyle, alkyle, hydroxyalkyle ou fluoroalkyle, étant entendu que si R⁵ représente un atome de fluor ou un groupe hydroxyle, R⁴ doit être un atome d'hydrogène ou un groupe alkyle, R⁵ représente un atome d'hydrogène ou de fluor, ou un groupe hydroxyle, alkyle, hydroxyalkyle ou fluoroalkyle, ou bien R⁴ et R⁵ pris ensemble représentent un atome d'oxygène doublement lié, R⁶ et R⁷ pris ensemble forment une deuxième liaison carbone-carbone, R⁸ peut être un atome d'hydrogène ou un groupe CH₃, et n est un nombre entier et a une valeur de 1 à 5, l'un quelconque des atomes de carbone en positions 20, 22 et 23 sur la chaîne latérale pouvant être remplacé par un atome d'oxygène, de soufre ou d'azote.

10. Utilisation selon la revendication 9, dans laquelle le composé est la 1,25-dihydroxyvitamine D₃, la 19-nor-1,25-dihydroxyvitamine D₂, la 19-nor-21-épi-1,25-dihydroxyvitamine D₃, la 1,25-dihydroxy-24-homo-22-dihydro-22E-vitamine D₃, ou la 19-nor-1,25-dihydroxy-24-homo-22-dihydro-22E-vitamine D₃.

## Patentansprüche

1. Verwendung einer Vitamin D-Verbindung bei der Herstellung eines Arzneimittels zur Behandlung von chronischer Allotransplantat-Nephropathie bei einem Nieren-Transplantationspatienten, wobei der Transplantationspatient einer immunosuppressiven Therapie unterliegt.

2. Verwendung nach Anspruch 1, wobei es sich bei der Vitamin D-Verbindung um 1,25-Dihydroxyvitamin D₃ handelt.

3. Verwendung nach Anspruch 1, wobei es sich bei der Vitamin D-Verbindung um eine 1α-Hydroxyverbindung handelt.

4. Verwendung nach Anspruch 1, wobei die Menge der verabreichten Vitamin D-Verbindung 0,1 µg bis 50 µg pro Tag pro 72,6 kg (160 1b)-Patient beträgt.

5. Verwendung nach Anspruch 4, wobei die Menge der verabreichten Vitamin D-Verbindung 0,1 µg bis 0,75 µg pro Tag pro 72,6 kg (160 1b)-Patient beträgt.

6. Verwendung nach Anspruch 1 oder 2, wobei die Vitamin D-Verbindung zur oralen und täglichen Verabreichung geeignet ist.

7. Verwendung nach Anspruch 1, wobei die Verabreichung der Vitamin D-Verbindung innerhalb von 24 Stunden nach dem Nieren-Transplantationsvorgang beginnt.

8. Verwendung nach Anspruch 1, wobei die Verabreichung der Vitamin D-Verbindung vor dem Nieren-Transplantationsvorgang beginnt.

9. Verwendung nach Anspruch 1, wobei die Vitamin D-Verbindung die folgende Formel aufweist: 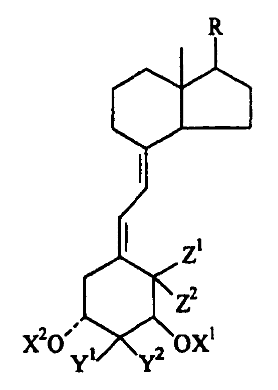
wobei X¹ und X² jeweils aus Wasserstoff und Acyl ausgewählt sind;
wobei Y¹ und Y² Wasserstoff bedeuten können oder einer dieser Reste 0-Aryl, O-Alkyl, Aryl, Alkyl mit 1-4 Kohlenstoffatomen bedeuten kann, oder diese Reste zusammen ein Alken der Strukturformel 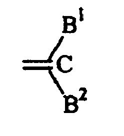
bilden, wobei B¹ und B² aus H, Alkyl mit 1-4 Kohlenstoffatomen und Aryl ausgewählt sind, oder Alkyl eine β- oder α-Konfiguration aufweisen kann;
Z¹=Z²=H oder Z¹ und Z² zusammen die Bedeutung =CH₂ haben; und
wobei R eine Alkyl-, Hydroxyalkyl oder Fluoralkylgruppe bedeutet oder R die folgende Seitenkette bedeuten kann 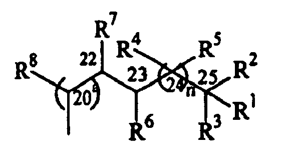
wobei (a) eine S- oder R-Konfiguration aufweisen kann, R¹ Wasserstoff, Hydroxy oder O-Acyl bedeutet, R² und R³ aus Alkyl, Hydroxyalkyl und Fluoralkyl ausgewählt sind oder zusammen die Gruppe -(CH₂)ₘ- bedeuten, wobei m eine ganze Zahl mit einem Wert von 2 bis 5 ist, R⁴ aus Wasserstoff, Hydroxy, Fluor, 0-Acyl, Alkyl, Hydroxyalkyl und Fluoralkyl ausgewählt ist, wobei dann, wenn R⁵ Hydroxy oder Fluor bedeutet, R⁴ Wasserstoff oder Alkyl sein muss, R⁵ aus Wasserstoff, Hydroxy, Fluor, Alkyl, Hydroxyalkyl und Fluoralkyl ausgewählt ist oder R⁴ und R⁵ zusammen doppelt gebundenen Sauerstoff bedeuten, R⁶ und R⁷ zusammen eine Kohlenstoff-Kohlenstoff-Doppelbindung bedeuten, R⁸ H oder CH₃ sein kann und wobei n eine ganze Zahl mit einem Wert von 1 bis 5 ist und wobei der Kohlenstoff in einer der Positionen 20, 22 oder 23 in der Seitenkette durch ein O-, S- oder N-Atom ersetzt sein kann.

10. Verwendung nach Anspruch 9, wobei die Verbindung ausgewählt ist aus 1,25-Dihydroxyvitamin D₃, 19-nor-1,25-Dihydroxyvitamin D₂, 19-nor-21-epi-1,25-Dihydroxyvitamin D₃, 1,25-Dihydroxy-24-homo-22-dehydro-22E-vitamin D₃ und 19-nor-1,25-Dihydroxy-24-homo-22-dehydro-22E-vitamin D₃.
